# EUROPEAN PATENT APPLICATION

(11) **EP 1 480 251 A2**
(43) Date of publication of application: **24.11.2004**
(21) Application number: 04252996.6
(22) Date of filing: 21.05.2004
(51) Int. Cl.: H01J 49/04

(54) **System of analyzing complex mixtures of biological and other fluids to identify biological state information**

(30) Priority: 22.05.2003 US 473272 P; 20.08.2003 US 645863; 16.01.2004 US 760100
(71) Applicant: Predicant Biosciences, Inc., South San Francisco, CA 94080 (US)
(72) Inventor: Heller, Jonathan C., San Francisco, CA 94107 (US); Dahl, Carol A., Mercer Island, WA 98040 (US); Stults, John T., Redwood City, CA 94062 (US); Foley, Peter, Los Altos Hills, CA 94022 (US); Ellsworth, Stoughton L. Jr., Palo Alto, CA 94301 (US); Andell, Frank III, Berkeley, CA 94704 (US); Greenquist, Alfred, San Jose, Ca 95130 (US)
(74) Representative: Bizley, Richard Edward

(57) **Abstract**

A method for use in classifying patient samples. The method includes steps of collecting case samples representing a clinical phenotypic state and control samples representing patients without said clinical phenotypic state. Preferably the system uses a mass spectrometry platform system to identify patterns of polypeptides in said case samples and in the control samples without regard to the specific identity of at least some of said polypeptides. Based on identified representative patterns of the state, the system performs optimized assays for greater sensitivity, specificity, and/or cost effectiveness and the method provides and allows for the marketing of diagnostic products using representative patterns.

## Description

### CROSS REFERENCE TO RELATED APPLICATIONS

This application claims priority to U.S. Patent Application Serial Number 10/645,863 filed August 20, 2003, which claimed the benefit of U.S. Provisional Application Serial Number 60/473,272 filed May 22, 2003, and U.S. Patent Application Serial Number 10/760,100 filed January 16, 2004, which is a Continuation-in-Part of U.S. Patent Application Serial Number 60/473,272. Such applications are incorporated by reference for all purposes.

### BACKGROUND OF THE INVENTION

A common aspect of all life on earth is the use of polypeptides as functional building blocks and the encryption of the instructions for the building blocks in the blueprint of nucleic acids (DNA, RNA). What distinguishes between living entities lies in the instructions encoded in the nucleic acids of the genome and the way the genome manifests itself in response to the environment as proteins. The complement of proteins, protein fragments, and peptides present at any specific moment in time defines who and what we are at that moment, as well as our state of health or disease.

One of the greatest challenges facing biomedical research and medicine is the limited ability to distinguish between specific biological states. This is reflected in the limited ability to detect the earliest stages of disease, anticipate the path any apparent disease will take in one patient versus another, predict the likelihood of response for any individual to a particular treatment, and preempt the possible adverse affects of treatments on a particular individual.

New technologies and strategies are needed to inform medical care and improve the repertoire of medical tools, as well as business methods to utilize such technologies and strategies.

### BRIEF SUMMARY OF THE INVENTION

According to one embodiment of the invention, a method is provided that includes the steps of collecting more than 10 case samples representing a clinical phenotypic state and more than 10 control samples representing patients without said clinical phenotypic state; using a mass spectrometry platform system to input said case samples and said control samples, identify patterns of polypeptides in said case samples and in said control samples without regard to the specific identity of at least some of said proteins; identifying representative patterns of the phenotypic state; and providing diagnostic products using said representative patterns. Such patterns contain preferably more than 15 polypeptides that are represented on output of said mass spectrometer, but the identity of at least some of said more than 15 polypeptides may not be known.

### BRIEF DESCRIPTION OF THE FIGURES

Figure 1 is an overall flowchart illustrating the operation of one embodiment of the methods of the present invention.

Figure 2 is a diagram illustrating preferred aspects of the invention herein.

### DETAILED DESCRIPTION OF THE INVENTION

The methods herein utilize and apply a system that is able to differentiate biological states with reliability, reproducibility, and sensitivity. In one embodiment, the system relies on an integrated, reproducible, sample preparation, separation, and electrospray ionization system in a microfluidics format, with high sensitivity mass spectrometry and informatics. This system will serve as the foundation for the discovery of patterns of polypeptides or other biological markers that reflect and differentiate biological states specific for various states of health and disease. For the purposes herein, the term "polypeptides" includes, for example proteins, peptides, and/or protein fragments. These patterns of polypeptides that reflect and differentiate biological states will then be utilized in clinically useful formats and in research contexts. Clinical applications will include detection of disease; distinguishing disease states to inform prognosis, selection of therapy, and the prediction of therapeutic response; disease staging; identification of disease processes; prediction of efficacy; prediction of adverse response; monitoring of therapy associated efficacy and toxicity; and detection of recurrence.

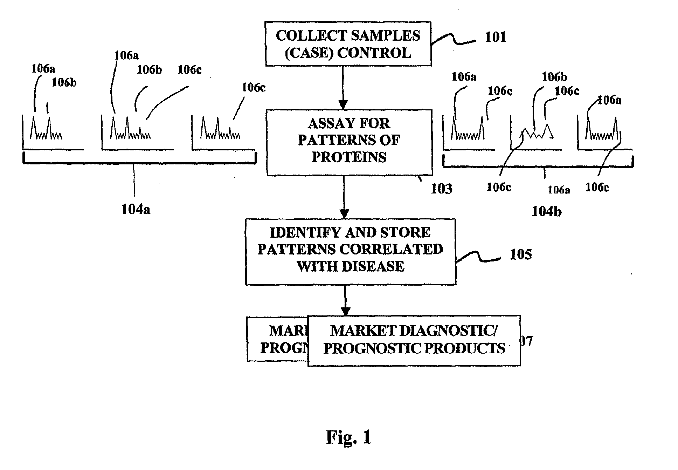

Figure 1 illustrates the overall process of the methods disclosed herein. At step 101 the involved business (alone or with collaborators) collects a representative sample set of case samples and control samples. The case samples will be those wherein a patient exhibits a particular disease state or other phenotype. For example, the case samples may be those where a patient exhibits a response to a drug. Conversely, the control samples will be collected from patients that do not exhibit the phenotype under study, such as those that do not have the disease or response to a drug. Preferably more than 10 case and 10 control samples are collected for use in identifying protein signals of interest. Preferably more than 20 case and 20 control samples, preferably more than 50 case and 50 control samples, preferably more than 100 case and 100 control samples, and most preferably more than 500 case and 500 control samples are collected.

At step 103 the case and control samples are assayed to identify patterns of markers that are present in the case and control samples. In preferred embodiments the markers are polypeptides such as proteins, although they may also include small molecules, nucleic acids, polysaccharides, metabolites, lipids, or the like. Preferably, the patterns are obtained without advance selection or screening of the particular polypeptides involved. In some embodiments the patterns are obtained without identification of some or all of the markers that are shown in the pattern. Three conceptual patterns are illustrated for cases at 104a and controls at 104b. As shown, the patterns are greatly simplified from those that will be actually observed.

Preferably the assay identifies the presence of more than 100 polypeptides, preferably more than 200 polypeptides, more preferably more than 500 polypeptides, more preferably more than 1000 polypeptides, and more preferably more than 2000 polypeptides. While the identity of some of the polypeptides will be known from prior studies, it is not necessary to specifically identify all of the polypeptides indicated by the assay. Instead, the present invention takes advantage of the presence of (or absence of) a pattern of many polypeptides repeatedly found to be in the cases in a pattern distinct from the controls to study phenotypes and/or for diagnostics. In various embodiments a number of polypeptides are represented in the pattern, but the identity of some of these polypeptides may not be known. For example, more than 15 polypeptides can be represented, more than 30 polypeptides can be represented, more than 50 polypeptides can be represented, more than 100 polypeptides can be represented, and more than 1000 polypeptides can be represented.

In preferred embodiments, the invention relies on a mass spectrometry system to perform the assays. Preferably such systems allow for the capture and measure of most or all of the instances of a polypeptide in a sample that is introduced in the mass spectrometer for analysis. Using such systems it is preferable that one can observe those polypeptides with high information-content but that are only present at low concentrations, such as those "leaked" from diseased tissue. Other high information-content polypeptides may be those that are related to the disease, for instance, those that are generated in the tumor-host environment.

In some embodiments, an early assay, such as the first assay, is followed by a later assay. The early assay normally will be used in initial identification of the polypeptides that identify or separate cases from controls. The later assay is adjusted according to parameters that can focus diagnostics or evaluation of regions of interest, such as regions of high variability, i.e. those regions or markers where there are significant differences between case samples and control samples. The parameters can be determined, for example, by an early assay which may identify the regions of interest, which may be on one technology platform, and by a later assay on the same or a different platform.

At step 105 bioinformatics systems are utilized to identify the differences in the polypeptide patterns in the case and control samples. Such techniques may be proceeded by various data cleanup steps. Patterns will be composed of the relative representation of numerous polypeptides or other biological entities, the collective profile of which will be more important than the presence or absence of any specific entities. By identifying patterns in blood or other patient samples, the methods will not only provide the window to the presence of disease and other pathology in some embodiments, but also to the ongoing response to the disease or pathologic condition in other embodiments. In a high throughput mode, data from a first sample are evaluated in a bioinformatics system at the same time another sample is being processed in, for example, a mass spectrometry system.

As shown in the three simplified patterns for "cases" 104a, peaks 106a and 106b tend to be observed in three "case" samples at higher levels. Conversely, less or no signal is observed at peak 106c in the three case samples. By contrast, in the control samples 104b, peaks 106a and 106c tend to be observed while peak 106b tends to be at low levels. Of course, the patterns shown in Fig. 1 are greatly simplified, and there will be much more complex patterns in actual practice, such as tens, hundreds, or thousands of such peaks. In the particular example illustrated in Fig. 1, peak 106a is not informative, while peak 106b tends to occur in cases, and peak 106c tends to occur in controls. Automated systems will generally be applied in the identification of the patterns that distinguish cases and controls. The measurement of patterns of multiple signals will enable the identification of subtle differences in biological state and make the identification of that state more robust and less subject to biological noise.

At step 107 the business applying the method uses the patterns of polypeptides present in the sample to identify the disease state of a patient sample in, for example, a diagnostic setting. Samples used in both the steps 101 and 107 will, in preferred embodiments be serum samples, although tissue or bodily fluid samples from a variety of sources will be used in alternative embodiments. Preferably, though not necessarily, the system used in the diagnostic application is based upon the same technology platform as the platform used to identify the patterns in the first instance. For example, if the platform used to identify the patterns in the first instance is a time of flight mass (TOF) spectrometer, it is preferred that the diagnostic applications of the patterns are run on a time of flight mass spectrometer.

The marketing of the products can take a number of forms. For example, it may be that the developer actually markets the instruments and assays into the diagnostic research market. In alternative embodiments, the developer of the patterns will partner with, for example, a large diagnostic company that will market those products made by the developer, alone or in combination with their own products. In alternative embodiments, the developer of the patterns licenses the intellectual property in the patterns to a third party and derives revenue from licensing income arising from the pattern information.

The method herein can obtain revenue by various means, which may vary over time. Such sources may include direct sale revenue of products, including revenue from diagnostic assays or services, upfront license fees, research payment fees, milestone payments (such as upon achievement of sales goals or regulatory filings), database subscription fees, and downstream royalties and from various sources including government agencies, academic institution and universities, biotechnology and pharmaceutical companies, insurance companies, and health care providers.

Often, diagnostic services hereunder will be offered by clinical reference laboratories or by way of the sale of diagnostic kits. Clinical reference laboratories generally process large number of patient samples on behalf of a number of care givers and/or pharmaceutical companies. Such reference laboratories in the United States are normally qualified under CLIA and/or CAP regulations, and qualification varies country by country in the EU. Of course, other methods may also be used for marketing and sales such as direct sales of kits approved by FDA or equivalent approved products or products with CE Marks under the In Vitro Diagnostics Directive. In some cases the developer of the pattern content will license the intellectual property and/or sell kits and/or reagents to a reference laboratory that will combine them with other reagents and/or instruments in providing a service.

In the short term, the methods disclosed generate revenue by, for example, providing application specific research or diagnostic services to third parties to discover and/or market the patterns. Examples of third-parties include customers who purchase diagnostic or research products (or services for discovery of patterns), licensees who license rights to pattern recognition databases, and partners who provide samples in exchange for downstream royalty rights and/or up front payments from pattern recognition. Depending on the fee, diagnostic services may be provided on an exclusive or non-exclusive basis.

Revenue can also be generated by entering into exclusive and/or non-exclusive contracts to provide polypeptide profiling of patients and populations. For example, a company entering clinical trials may wish to stratify a patient population according to, for example, drug regimen, effective dosage, or otherwise. Stratifying a patient population may increase the efficacy of clinical trial (by removing, for example, non responders), thus allowing the company to enter into the market sooner or allow a drug to be marketed with a diagnostic test that identifies patients that may have an adverse response or be non-responsive. In addition, insurance companies may wish to obtain a polypeptide profile of a potential insured and/or to determine if, for example a drug or treatment will be effective for a patient.

In the long term, revenue may be generated by alternative methods. For example, revenue can be generated by entering into exclusive and/or non-exclusive drug discovery contracts with drug companies (e.g., biotechnology companies and pharmaceutical companies). Such contracts can provide for downstream royalties on a drug based on the identification or verification of drug targets (e.g., a particular protein or set of polypeptides associated with a phenotypic state of interest), or on the identification of a subpopulation in which such drug should be utilized. Alternatively, revenue may come from a licensee fee on a diagnostic itself. The diagnostic services, patterns, and tools herein can further be provided to a pharmaceutical company in exchange for milestone payments or downstream royalties. Revenue may also be generated from the sale of disposable fluidics devices, disposable microfluidics devices, or other assay reagents or devices in for example the research market, diagnostic market, or in clinical reference laboratories. Revenue may also be generated from licensing of applications-specific software or databases. Revenue may, still further, be generated based on royalties from technology platform providers who may license some or all of the proprietary technology. For example, a mass-spectrometer platform provider may license the right to further distribute software and computer tools and/or polypeptide patterns.

In preferred embodiments, the TOF device utilized herein is coupled to a microfluidic separations device. The sample preparation techniques preferably concentrate the polypeptides the mass spectrometer is best able to detect and/or are which are most informative, and deplete the ones that are more difficult to detect and/or are less informative (because, for example, they appear in both case and control samples). In most preferred embodiments the microfluidic separations device is a disposable device that is readily attached to and removed from the TOF mass spectrometer, and sold as a disposable, thereby providing a recurring revenue stream to the involved business. Preferably, a mass spectrometer is utilized that will accept a continuous sample stream for analysis and provide high sensitivity throughout the detection process.

Sample preparation will, in some embodiments, include the removal of high abundance polypeptides, removal of polypeptides expected to be in abundance in all samples, addition of preservatives and calibrants, and desalting. These steps will allow sensitive measurement of concentrations of information-rich polypeptides, such as those that have leaked from tissue, as compared to polypeptides that would carry little information, such as those highly abundant and native to serum. Prepared samples will then be separated using fast molecular separations methods with high peak capacities. An electrospray ionization (ESI) interface may be integrated on the microfluidics chip, which will ionize and spray the prepared and separated serum directly into a mass spectrometer and is preferably sold as part of a disposable component to assure high reliability of the system. The microfluidics-based separations preferably provide the polypeptide mixtures at flow rates and at complexity levels that are matched to the mass spectrometer's optimal performance regions. The mass spectrometer's sensitivity is preferably optimized to detect the species most likely to differentiate biological states. Preferably, the reagents necessary for performing these steps are provided in or along with the microfluidics device, thereby allowing for additional recurring revenue to the involved business and higher performance for the user.

The sample preparation system will provide for different operations depending upon the detection device to be utilized. The sample preparation system preferably provides for protein denaturation prior to processing on the mass spectrometer. Analytes of interest herein will in some cases be in a protein bound form. Preferably the system provides for denaturation of proteins preferably prior to the removal of high abundance materials (such as albumin or other proteins from serum or plasma samples). By denaturing such proteins prior to their removal, bound analytes of interest will be released such that they can be meaningful in later analysis. Denaturation may utilize any of several techniques including the use of heat, high salt concentrations, the use of acids, base, chaotropic agents, organic solvents, detergents and/or reducing agents. Liotta, Lance, A., et al., "Written in Blood," *Nature* (10/30/2003), Volume 425, page 905. Tirumalai, Radhakrishna S., et al. "Characterization of the Low Molecular Weight Human Serum Proteome," *Molecular & Cellular Proteomics 2.10* (8/13/2003), pages 1096-1103.

The system used for removal of high abundance polypeptides may be based on, for example, the use of high affinity reagents for removal of the polypeptides, the use of high molecular weight filters, ultracentrifugation, precipitation, and/or electrodialysis. Polypeptides that will often be removed will include, for example, those involved in normal metabolism, and a wide variety of other indications not of relevance to a particular assay. Such proteins may be removed through, for example, a solid phase extraction resin. Additionally, the system may include a reversed phase chromatography device, for example, for separation of small molecules and/or to trap, desalt, and separate a protein mixture.

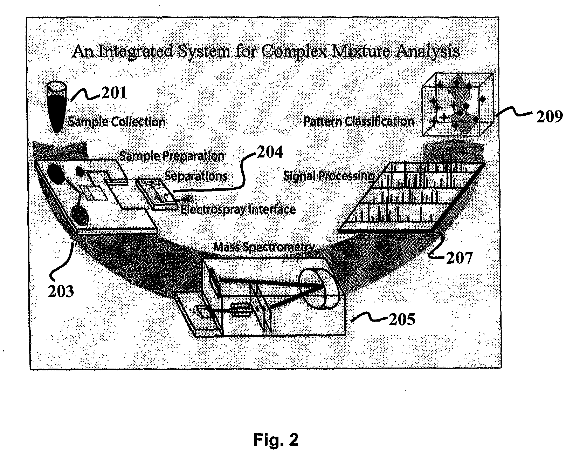

Figure 2 illustrates additional aspects of an exemplary system platform used herein, which may be utilized in both the applications of studying protein patterns that distinguish case and control samples, and/or in using patterns to diagnose individuals. The invention involves an integrated system to a) discover and b) assay patterns of polypeptides that reflect and differentiate biological and clinical states of organisms, including patients, in biological materials including but not limited to body fluids. Biological and clinical states include but are not limited to states of development; age; health; pathology; disease detection, process, or staging; infection; toxicity; or response to chemical, environmental, or drug factors (such as drug response phenotyping, drug toxicity phenotyping, or drug effectiveness phenotyping). Biological fluids 201 include but are not limited to serum, plasma, whole blood, nipple aspirate, pancreatic fluid, trabecular fluid, lung lavage, urine, cerebrospinal fluid, saliva, sweat, pericrevicular fluid, and tears.

The system provides for the integration of fast molecular separations and electrospray ionization system 204 on a microfluidics platform 203. The system provides processed samples to a high sensitivity time of flight mass spectrometer 205. Signal processing system and pattern extraction and recognition tools 205 incorporate domain knowledge to extract information from polypeptide patterns and classify the patterns to provide a classification 209. The signal processing system may include or be coupled to other software elements as well. For example, the signal processing system may provide for an easy to use user interface on the associated computer system and/or a patient database for integration of results into an institution's laboratory or patient information database system.

The microfluidics device(s) 203 may be formed in plastic by means of etching, machining, cutting, molding, casting or embossing. The microfluidics device(s) may be made from glass or silicon by means of etching, machining, or cutting. The device may be formed by polymerization on a form or other mold. The molecular separations unit or the integrated fast molecular separations/electrospray ionization unit may provide additional sample preparation steps, including sample loading, sample concentration, removal of salts and other compounds that may interfere with electrospray ionization, removal of highly abundant species, concentration of the sample to a smaller volume, proteolytic or chemical cleavage of components within the biological material, and/or aliquoting into storage containers. The particular operations performed by the device will depend upon the detection technology that is utilized.

The device(s) for separations and electrospray may be either single use for a single sample, multi-use for a single sample at a time with serial loading, single use with parallel multiple sample processing, multi-use with parallel multiple sample processing or a combination. Separations processes may include isoelectric focusing, electrophoresis, chromatography, or electrochromatography. The separations device may include collection areas or entities for some or all of the purified or partially purified fractions.

it is to be understood that the inventions herein are illustrated primarily with regard to mass spectrometry as a detection device, but other devices may be used alone or with the mass spectrometer. For example, detection devices may include electrochemical, spectroscopic, or luminescent detectors, and may be integral with the microfluidics device.

Mass spectrometers that may be used include quadrupole, ion trap, magnetic sector, Fourier transform ion cyclotron resonance instruments, or an orthogonal time-of-flight mass spectrometer which includes an analyzer that receives an ion beam from an electrospray ionization (ESI) source.

In preferred embodiments the system also adapts the speed of the system in response to the detection of known markers that are likely to be present in all samples, and which are readily detectable. Since separations will often vary in retention or migration time, by detecting molecules that are known, likely to be in all samples, and easily detectable, and then comparing the speed at which they have passed through the system in comparison to a standard from other experiments, it becomes possible to speed the system up by speeding the separations in response to the detection of slower than expected migration time, or slowing the system down in response to faster than expected migration times. The speed may be adjusted through, for example, adjustments in system pressure, voltage, current flow, or temperature. Preferably, the system is operated faster or slower by changing the voltage. Representative peptides and proteins that could be spiked into samples and could be used for this purpose include neurotensin, lysozyme, aprotinin, insulin b-chain, and renin substrate. In addition, the speed of operation of the device may be slowed to provide greater accuracy in the detection of molecules of particular interest in a spectrum. Conversely, the system may be operated more quickly during the times when components of low interest would be expected to be detected.

In some embodiments pressure is added to move the components through the electrophoretic device, especially to migrate components to the end of an electrophoretic separation capillary (in conjunction with the use of the electro osmotic flow). The pressure produces buffer flow that is required to maintain a stable electrospray.

Ions formed by electrospray ionization will normally be chiefly singly or multiply charged ions of molecules, with charge coming from protons or alkali metal bound to the molecules. Ion excitation may be produced by collision of ions with background gas or an introduced collision gas. Horn, D.M., et at., "Activated ion electron capture dissociation for mass spectral sequencing of larger (42 kDa) proteins," *Anal. Chem.* (2000), Volume 72, 4778-84. Doroshenko, V.M., Cotter, R.J. "High-performance collision-induced dissociation of peptide ions formed by matrix-assisted laser desorption/ionization in a quadrupole ion trap mass spectrometer," *Anal. Chem.* (1995), Volume 67, 2180-7. Alternatively, excitation may be from collision with other ions, a surface, interaction with photons, heat, electrons, or alpha particles. Through excitation of the sample in an electrospray the information content of the process should be altered and/or enhanced. Such excitation may, for example, desolvate ions, dissociate noncovalently bound molecules from analyte ions, break up solvent clusters, fragment background ions to change their mass to charge ratio and move them to a ratio that may interfere less with the analysis, strip protons and other charge carriers such that multiply charged ions move to different regions of the spectrum, and fragment analyte ions to produce additional, more specific or sequence-related information.

In preferred embodiments the excitation system may be turned on and off to obtain a set of spectra in both states. The information content of the two spectra will, in most cases, be far greater than the information content of either single spectra. In such embodiments the system will include a switching device for activating and de-activating the excitation/ionization system. Analysis software will be configured in this case to analyze the sample separately both in the "on" state of the excitation system and in the "off" state of the excitation system. Different markers may be detected more efficiently in one or the other of these two states.

### Sample Collection

Case samples are obtained from individuals with a particular phenotypic state of interest. Examples of phenotypic states include, phenotypes resulting from an altered environment, drug treatment, genetic manipulations or mutations, injury, change in diet, aging, or any other characteristic(s) of a single organism or a class or subclass of organisms. In a preferred embodiment, a phenotypic state of interest is a clinically diagnosed disease state. Such disease states include, for example, cancer, cardiovascular disease, inflammatory disease, and infectious disease. Control samples are obtained from individuals who do not exhibit the phenotypic state of interest or disease state (e.g., an individual who is not affected by a disease or who does not experience negative side effects in response to a given drug). Alternatively, states of health can be analyzed.

Cancer phenotypes are studied in some aspects of the method. Examples of cancer include, but are not limited to, breast cancer, skin cancer, bone cancer, prostate cancer, liver cancer, lung cancer, brain cancer, cancer of the larynx, gallbladder, pancreas, rectum, parathyroid, thyroid, adrenal, neural tissue, head and neck, colon, stomach, bronchi, kidneys, basal cell carcinoma, squamous cell carcinoma of both ulcerating and papillary type, metastatic skin carcinoma, osteo sarcoma, Ewing's sarcoma, veticulum cell sarcoma, myeloma, giant cell tumor, small-cell lung tumor, gallstones, islet cell tumor, primary brain tumor, acute and chronic lymphocytic and granulocytic tumors, hairy-cell tumor, adenoma, hyperplasia, medullary carcinoma, pheochromocytoma, mucosal neuronms, intestinal ganglloneuromas, hyperplastic corneal nerve tumor, marfanoid habitus tumor, Wilm's tumor, seminoma, ovarian tumor, leiomyomater tumor, cervical dysplasia and in situ carcinoma, neuroblastoma, retinoblastoma, soft tissue sarcoma, malignant carcinoid, topical skin lesion, mycosis fungoide, rhabdomyosarcoma, Kaposi's sarcoma, osteogenic and other sarcoma, malignant hypercalcemia, renal cell tumor, polycythermia vera, adenocarcinoma, glioblastoma multiforma, leukemias, lymphomas, malignant melanomas, epidermoid carcinomas, and other carcinomas and sarcomas.

Pregnancy related disorders that may be investigated herein include pre-eclampsia, eclampsia pre-term birth, growth restriction in utero, rhesus incompartability, retained placenta, septicemia, separation of the placenta, ectopic pregnancy, hypermosis gravidarum, placenta previa, erythroblastosis fetalis, pruritic urticarial papula and plaques.

Cardivascular disease may be studied in other applications of the invention. Examples of cardiovascular disease include, but are not limited to, congestive heart failure, high blood pressure, arrhythmias, cholesterol, Wolff-Parkinson-White Syndrome, long QT syndrome, angina pectoris, tachycardia, bradycardia, atrial fibrillation, ventricular fibrillation, congestive heart failure, myocardial ischemia, myocardial infarction, cardiac tamponade, myocarditis, pericarditis, arrhythmogenic right ventricular dysplasia, hypertrophic cardiomyopathy, Williams syndrome, heart valve diseases, endocarditis, bacterial, pulmonary atresia, aortic valve stenosis, Raynaud's disease, cholesterol embolism, Wallenberg syndrome, Hippel-Lindau disease, and telangiectasis.

Inflammatory disease may be studied in other applications of the method. Examples of inflammatory disease include, but are not limited to, rheumatoid, arthritis, non-specific arthritis, inflammatory disease of the larynx, inflammatory bowel disorder, pelvic inflammatory disease, inflammatory disease of the central nervous system, temporal arteritis, polymyalgia rheumatica, ankylosing spondylitis, polyarteritis nodosa, Reiter's syndrome, scleroderma, systemis lupus and erythematosus.

Infectious disease may be studied in still further aspects of the method. Examples of infectious disease include, but are not limited to, AIDS, hepatitis C, SARS, tuberculosis, sexually transmitted diseases, leprosay, lyme disease, malaria, measles, meningitis, mononucleosis, whooping cough, yellow fever, tetanus, arboviral encephalitis, and other bacterial, viral, fungal or helminthic diseases.

Samples may be collected from a variety of sources in a given patient depending on the application of the business. In some embodiments samples are collected on the account of the company itself, while in other examples they are collected in collaboration with an academic collaborator or pharmaceutical collaborator that, for example, is collecting samples in a clinical trial. Samples collected are preferably bodily fluids such as blood, serum, sputum, including, saliva, plasma, nipple aspirants, synovial fluids, cerebrospinal fluids, sweat, urine, fecal matter, pancreatic fluid, trabecular fluid, cerebrospinal fluid, tears, bronchial lavage, swabbings, bronchial aspirants, semen, precervicular fluid, vaginal fluids, pre-ejaculate, etc. In a preferred embodiment, a sample collected is approximately 1 to 5 ml of blood.

In some instances, samples may be collected from individuals over a longitudinal period of time (e.g., once a day, once a week, once a month, biannually or annually). Obtaining numerous samples from an individual over a period of time can be used to verify results from earlier detections and/or to identify an alteration in polypeptide pattern as a result of, for example, aging, drug treatment, pathology, etc. Samples can be obtained from humans or non-humans. In a preferred embodiment, samples are obtained from humans.

In some instances, samples may be collected from individuals over a longitudinal period of time (e.g., once a day, once a week, once a month, biannually or annually). The longitudinal period may, for example, also be before, during, and after a stress test or a drug treatment. Obtaining numerous samples from an individual over a period of time can be used to verify results from earlier detections and/or to identify an alteration in polypeptide pattern as a result of, for example, aging, drug treatment, pathology, etc. Samples can be obtained from humans or non-humans. In a preferred embodiment, samples are obtained from humans.

Sample preparation and separation can involve any of the following procedures, depending on the type of sample collected and/or types of protein searched: removal of high abundance polypeptides (e.g., albumin, and transferrin); addition of preservatives and calibrants, denaturation, desalting of samples; concentration of sample polypeptides; protein digestions; and fraction collection. Preferably, sample preparation techniques concentrate information-rich polypeptides (e.g., polypeptides that have "leaked" from diseased cells or are produced by the host response to the tumor) and deplete polypeptides that would carry little or no information such as those that are highly abundant.

Sample preparation can take place in a manifold or preparation/separation device. In preferred embodiment, such preparation/separation device is a microfluidics device. Optimally, the preparation/separation device interfaces directly or indirectly with a detection device. In another embodiment, such preparation/separation device is a fluidics device.

Approximately 100 µL of a sample is analyzed per assay in some particular embodiments of the invention. Removal of undesired polypeptides (e.g., high abundance, uninformative, or undetectable polypeptides) can be achieved using high affinity reagents, high molecular weight filters, untracentrifugation and/or electrodialysis. High affinity reagents include antibodies that selectively bind to high abundance polypeptides or reagents that have a specific pH, ionic value, or detergent strength. High molecular weight filters include membranes that separate molecules on the basis of size and molecular weight. Such filters may further employ reverse osmosis, nanofiltration, ultrafiltration and microfiltration.

Ultracentrifugation is another method for removing undesired polypeptides. Ultracentrifugation is the centrifugation of a sample at about 60,000 rpm while monitoring with an optical system the sedimentation (or lack thereof) of particles. Finally, electrodialysis is an electromembrane process in which ions are transported through ion permeable membranes from one solution to another under the influence of a potential gradient. Since the membranes used in electrodialysis have the ability to selectively transport ions having positive or negative charge and reject ions of the opposite charge, electrodialysis is useful for concentration, removal, or separation of electrolytes.

In a preferred embodiment, the manifold or microfluidics device performs electrodialysis to remove high molecular weight polypeptides or undesired polypeptides. Electrodialysis is first used to allow only molecules under approximately 30 kD (not a sharp cutoff) to pass through into a second chamber. A second membrane with a very small molecular weight (roughly 500 D) will allow smaller molecules to egress the second chamber.

After samples are prepared, polypeptides of interest may be separated. Separation can take place in the same location as the preparation or in another location. In a preferred embodiment, separation occurs in the same microfluidics device where preparation occurs, but in a different location on the device. Samples can be removed from an initial manifold location to a microfluidics device using various means, including an electric field. In preferred embodiment, the samples are concentrated during their migration to the microfluidics device using reverse phase beads and an organic solvent elution such as 50% methanol. This elutes the molecules into a channel or a well on a separation device of a microfluidics device. In another embodiment, samples are concentrated by isotachophoresis, in which ions are concentrated at a boundary between a leading and a trailing electrolyte of lower and higher electrophoretic mobilities, respectively.

Separation can involve any procedure known in the art, such as capillary electrophoresis (e.g., in capillary or on-chip) or chromatography (e.g., in capillary, column or on a chip).

Electrophoresis is the separation of ionic molecules such as polypeptides by differential migration patterns through a gel based on the size and ionic charge of the molecules in an electric field. Electrophoresis can be conducted in a gel, capillary or on a chip. Examples of gels used for electrophoresis include starch, acrylamide, agarose or combinations thereof. In a preferred embodiment, polyacrilamide gels are used. A gel can be modified by its cross-linking, addition of detergents, immobilization of enzymes or antibodies (affinity electrophoresis) or substrates (zymography) and pH gradient. Examples of capillaries used for electrophoresis include capillaries that interface with an electrospray.

Capillary electrophoresis (CE) is preferred for separating complex hydrophilic molecules and highly charged solutes. Advantages of CE include its use of small samples (sizes ranging from 1 to 10 ul), fast separation, easily reproducible, and the ability to be coupled to a mass spectrometer. CE technology, in general, relates to separation techniques that use narrow bore fused-silica capillaries to separate a complex array of large and small molecules. High voltages are used to separate molecules based on differences in charge, size and hydrophobicity. Depending on the types of capillary and buffers used, CE can be further segmented into separation techniques such as capillary zone electrophoresis (CZE), capillary isoelectric focusing (CIEF) and capillary electrochromatography (CEC).

Capillary zone electrophoresis (CZE), also known as free-solution CE (FSCE), is the simplest form of CE. The separation mechanism of CZE is based on differences in the charge-to-mass ratio of the analytes. Fundamental to CZE are homogeneity of the buffer solution and constant field strength throughout the length of the capillary. The separation relies principally on the pH-controlled dissociation of acidic groups on the solute or the protonation of basic functions on the solute.

Capillary isoelectric focusing (CIEF) allows amphoteric molecules, such as polypeptides, to be separated by electrophoresis in a pH gradient generated between the cathode and anode. A solute will migrate to a point where its net charge is zero. At this isoelectric point (the solute's pl), migration stops and the sample is focused into a tight zone. In CIEF, once a solute has focused at its pl, the zone is mobilized past the detector by either pressure or chemical means.

CEC is a hybrid technique between traditional liquid chromatography (HPLC) and CE. in essence, CE capillaries are packed with HPLC packing and a voltage is applied across the packed capillary, which generates an electro-osmotic flow (EOF). The EOF transports solutes along the capillary towards a detector. Both differential partitioning and electrophoretic migration of the solutes occurs during their transportation towards the detector, which leads to CEC separations. It is therefore possible to obtain unique separation selectivities using CEC compared to both HPLC and CE. The beneficial flow profile of EOF reduces flow related band broadening and separation efficiencies of several hundred thousand plates per meter are often obtained in CEC. CEC also makes it is possible to use small-diameter packings and achieve very high efficiencies.

Chromatography is another method for separating a subset of polypeptides. Chromatography is based on the differential absorption and elution of certain polypeptides. Liquid chromatography (LC), for example, involves the use of fluid carrier over a non-mobile phase. Conventional LC columns have an inner diameter of roughly 4.6 mm and a flow rate of roughly 1 ml/min. Micro-LC has an inner diameter of roughly 1.0 mm and a flow rate of roughly 40 ul/min. Capillary LC utilizes a capillary with an inner diameter of roughly 300 im and a flow rate of approximately 5 ul/min. Nano-LC is available with an inner diameter of 50 um -1 mm and flow rates of 200 nl/min. Nano-LC can vary in length (e.g., 5, 15, or 25 cm) and have typical packing of C18, 5 um particle size. In a preferred embodiment, nano-LC is used. Nano-LC provides increased sensitivity due to lower dilution of chromatographic sample. The sensitivity of nano-LC as compared to HPLC is approximately 3700 fold.

In preferred embodiments, the samples are separated on using capillary electrophoresis separation, more preferably CEC with sol-gels, or more preferably CZE. This will separate the molecules based on their eletrophoretic mobility at a given pH (or hydrophobicity in the case of CEC).

In other preferred embodiments, the steps of sample preparation and separation are combined using microfluidics technology. A microfluidic device is a device that can transport liquids including various reagents such as analytes and elutions between different locations using microchannel structures. Microfluidic devices provide advantageous miniaturization, automation and integration of a large number of different types of analytical operations. For example, continuous flow microfluidic devices have been developed that perform serial assays on extremely large numbers of different chemical compounds. Microfluidic devices may also provide the feature of disposability, to prevent sample carrying-over. By microfluidics device it is intended to mean herein devices with channels smaller than 1000 µm, preferably less than 500 µm, and more preferably less than 100 µm. Preferably such devices use sample volumes of less than 1000 µl, preferably less than 500 µl, and most preferably less than 100 µl.

In a preferred embodiment, microfluidic devices are composed of plastic and formed by means of etching, machining, cutting, molding, casting or embossing. The microfluidics devices may alternatively be made from glass or silicon by means of etching, machining, or cutting. The microfluidic devices may be either single use for a single sample; multi-use for a single sample at a time with serial loading; single use with parallel multiple sample processing; multi-use with parallel multiple sample processing; or a combination. Furthermore, more than one microfluidics device may be integrated into the system and interface with a single detection device.

Once prepared and separated, the polypeptides are automatically delivered to a detection device, which detects the polypeptides in a sample. In a preferred embodiment, polypeptides in elutions or solutions are delivered to a detection device by electrospray ionization (ESI). ESI operates by infusing a liquid containing the sample of interest through a channel or needle, which is kept at a potential (typically 3.5 kV). The voltage on the needle causes the spray to be charged as it is nebulized. The resultant droplets evaporate in a region maintained at a vacuum of several torr, until the solvent is essentially completely stripped off, leaving a charged ion. The charged ions are then detected by a detection device such as a mass spectrometer. In a more preferred embodiment, nanospray ionization (NSI) is used. Nanospray ionization is a miniaturized version of ESI and provides low detection limits using extremely limited volumes of sample fluid.

In preferred embodiments, separated polypeptides are directed down a channel that leads to an electrospray ionization emitter, which is built into a microfluidic device (an integrated ESI microfluidic device). Preferably, such integrated ESI microfluidic device provides the detection device with samples at flow rates and complexity levels that are optimal for detection. Such flow rates are, preferably, approximately 50-200 uL/min. Furthermore, a microfluidic device is preferably aligned with a detection device for optimal sample capture. For example, using dynamic feedback circuitry, a microfluidic device may allow for control positioning of an electrospray voltage and for the entire spray to be captured by the detection device orifice. The microfluidic device can be sold separately or in combination with other reagents, software tools and/or devices.

Calibrants can also be sprayed into detection device. Calibrants are used to set instrument parameters and for signal processing calibration purposes. Calibrants are preferably utilized before a real sample is assessed. Calibrants can interface with a detection device using the same or a separate interface as the samples. In a preferred embodiment, calibrants are sprayed into a detection device using a second interface (e.g., second spray tip).

### Polypeptide Detection

Detection devices can comprise of any device that is able to detect polypeptide presence and/or level, including for example, NMR, 2-D PAGE technology, Western blot technology, immuoanalysis technology and mass spectrometry. In a preferred embodiment, the model herein relies on a mass spectrometry to detect polypeptides present in a given sample. There are various forms of mass spectrometers that may be utilized by the methods of the present invention.

In a preferred embodiment, the method utilizes an ESI-MS detection device. An ESI-MS combines the novelty of ESI with mass spectrometry. Furthermore, an ESI-MS preferably utilizes a time-of-flight (TOF) mass spectrometry system. In TOF-MS, ions are generated by whatever ionization method is being employed and a voltage potential is applied. The potential extracts the ions from their source and accelerates them towards a detector. By measuring the time it takes the ions to travel a fixed distance, the mass of the ions can be calculated. TOF-MS can be set up to have an orthogonal-acceleration (OA). OA-TOF-MS may in some embodiments have better spectral resolution and duty cycle. OA-TOF-MS also has the ability to obtain spectra at a relatively high speed. Brock *et al.* Anal. Chem (1998) 70, 3735-41, discuss on-axis TOF known as Hadamard OA-TOF-MS. In addition to the MS systems disclosed above, other forms of EMI-MS include quadrupole mass spectrometry, ion trap mass spectrometry, and Fourier transform ion cyclotron resonance (FTICR-MS).

Quadrupole mass spectrometry consists of four parallel metal rods arranged in four quadrants (one rod in each quadrant). Two opposite rods have a positive applied potential and the other two rods have a negative potential. The applied voltages affect the trajectory of the ions traveling down the flight path. Only ions of a certain mass-to-charge ratio pass through the quadrupole filter and all other ions are thrown out of their original path. A mass spectrum is obtained by monitoring the ions passing through the quadrupole filter as the voltages on the rods are varied.

Ion trap mass spectrometry uses three electrodes to trap ions in a small volume. The mass analyzer consists of a ring electrode separating two hemispherical electrodes. A mass spectrum is obtained by changing the electrode voltages to eject the ions from the trap. The advantages of the ion-trap mass spectrometer include compact size, and the ability to trap and accumulate ions to increase the signal-to-noise ratio of a measurement

FTICR mass spectrometry is a mass spectrometric technique that is based upon an ion's motion in a magnetic field. Once an ion is formed, it eventually finds itself in the cell of the instrument, which is situated in a homogenous region of a large magnet. The ions are constrained in the XY plane by the magnetic field and undergo a circular orbit. The mass of the ion can now be determined based on the cyclotron frequency of the ion in the cell.

In a preferred embodiment, the model herein employs a TOP mass spectrometer, or more preferably, an ESI-TOF-MS, an OA-TOF-MS, or a multiplexed OA-TOF-MS (a multiplexed TOF mass spectrometer), or more preferably a mass spectrometer having a dual ion funnel to support dynamic switching between multiple quadrapoles in series, the second of which can be used to dynamically filter ions by mass in real time.

The detection device preferably interfaces with a separation/preparation device or microfluidic device, which allows for quick assaying of many of the polypeptides in a sample, or more preferably, most or all of the polypeptides in a sample. Preferably, a mass spectrometer is utilized that will accept a continuous sample stream for analysis and provide high sensitivity throughout the detection process (e.g., an ESI-MS). In another preferred embodiment, a mass spectrometer interfaces with one or more electrosprays, two or more electrosprays, three or more electrosprays or four or more electrosprays. Such electrosprays can originate from a single or multiple microfluidic devices.

The detection system utilized preferably allows for the capture and measurement of most or all of the polypeptides are introduced into the detection device. It is preferable that one can observe polypeptides with high information-content that are only present at low concentrations. By contrast, it is preferable to remove those in advance that are, for example, common to all cells, especially those in high abundance.

### Signal Processing/Pattern Recognition

The output from a detection device can then be processed, stored, and further analyzed or assayed using a bio-informatics system. A bio-informatics system can include one or more of the following: a computer; a plurality of computers connected to a network; a signal processing tool(s); a pattern recognition tool(s); and optionally a tool(s) to control flow rate for sample preparation, separation, and detection.

Data processing utilizes mathematical foundations. Generally, dynamic programming is preferably used to align a separation axis with a standard separation profile. Furthermore, intensities may be normalized, preferably by fitting roughly 90% of the intensity values into a standard spectrum. The data sets are then fitted using wavelets that are specifically designed for separation and mass spectrometer data. Data processing preferably filters out some of the noise and reduces spectrum dimensionality. This allows the business to identify the more highly predictive patterns.

In some embodiments, data processing may also involve the calibration of a mass-axis using linear correction determined by the calibrants. Calibration can take prior to any sample detection; after sample detection; or in recurring intervals, for example.

Following data processing, pattern recognition tools are utilized to identify subtle differences between phenotypic states. Pattern recognition tools are based on a combination of statistical and computer scientific approaches, which provide dimensionality reduction. Such tools are scalable.

### Examples

The following prophetic example illustrates certain aspects of the invention.

Approximately one to five ml of blood will be collected through venipuncture into special tubes that contain the appropriate calibrants/controls. Following thorough clot formation, serum will be isolated from sample following centrifugation. Serum sample will be aliquoted and frozen at -70°C until analysis. On the order of 100 uL of thawed sample will be placed in a disposable plastic device that fits into a manifold, and hereafter, the entire process would be automated. The device will perform electrodialysis on the sample. Using an electric field and tangential flow, the sample will be passed through a membrane that allows only molecules under approximately 30kD (not a sharp cutoff) to pass through into a second chamber. Molecules of with the opposite charge or large molecules will not pass. A second membrane with a very low molecular weight cutoff (∼500D) will allow small molecules to pass out of the second chamber. Molecules that remain in the second chamber will therefore be in a MW range (500D-30kD). Most of these molecules will be peptides, protein fragments and small proteins. Salts will have been removed, as will most of the abundant polypeptides, such as albumin. This process should take approximately 60 minutes.

The molecules of interest (i.e. those that remain in the second chamber) will then be moved to another location on the disposable device, again using an electric field, and onto reverse phase beads for sample concentration. Using an organic solvent elution such as 50% methanol, the molecules will be eluted into a channel or well on a second disposable device, this time a microfluidics chip. On this chip, a 1-5 minute capillary electrophoretic separation, CZE or CEC, will be run to separate the molecules on the basis of electrophoretic mobility at the given pH (or hydrophobicity in the case of CEC). Preferred separation peak widths under 1 second will be utilized.

Separated molecules will be directed down a channel that leads to a electrospray ionization emitter that is built onto each chip. Expected flow rates are 50-200 uL/min. Prior to starting the separation, the microfluidics device will be aligned with the mass spectrometer using dynamic feedback circuitry to optimally control positioning stage placement and electrospray voltage to establish a stable spray and, assuming appropriate nl flow rates, allow the entire spray to be captured in the mass spectrometer orifice. Standards/calibrants would also be sprayed into the mass spectrometer using a dedicated second spray tip and used to set instrument parameters and for signal processing calibration purposes before the real samples are run.

An orthogonal mass spectrometer captures the spray from the prepared/separated sample (given that it is separated, the molecules will be migrating in small groups) and yield a spectrum at a rate of 5 spectrum/s. The mass spectrometer incorporates a dual ion funnel to support dynamic switching between calibrants and analyte sprays to optimize instrument accuracy. The instrument contains multiple quadrapoles in series, the second of which can, in real time during a data acquisition run, be used to dynamically filter ions by mass, thus allowing increased dynamic range or focus on particular mass ranges of interest. The orthogonal implementation supportshigh throughput, high sensitivity, and high mass resolution.

A resulting data set from one sample would have on the order of 10⁹ data points. Each data set would take approximately 5 minutes to collect, from start to finish. While a data set is being analyzed, a second sample could be run through the system to increase throughput.

Each data set would have its mass axis calibrated through a linear correction determined by the calibrants run before the sample and by the calibrants run in parallel in the dual ion funnel. Then dynamic programming would be used to align the separations axis (using the TIC) to some standard separations profile. Intensities would then be normalized by fitting the 90% intensity values to a standard spectrum.

These corrected data sets would then be fit using wavelets (or vaguelettes) that are specifically designed for separations/mass spectrometer data. The parameterized information about the spectrum would be soft thresholded and otherwise filtered to both remove noise and reduce dimensionality.

During pattern discovery, a set of approximately 50 case and 50 controls of these filtered parameter sets would be entered into a pattern recognition tool such as a linear support vector machine, but probably multiple learning algorithms will be used on each data set. The space of tunable parameters for the learning machine will be searched, and optimal patterns that distinguish the sample classes will be found, as would be error bounds on that prediction using cross-validation.

During validation or in clinical assay, the filtered parameters from each new data set would be classified into a category by identifying which side of the decision boundary in the multidimensional parameter space that data set lies. Confidence intervals could also be calculated. This prediction and confidence interval would be reported back to the technician running the machine. In some embodiments the information about these clinical samples would be captured and those results and clinical outcomes of those patients in pattern recognition using more samples would be used, yielding better patterns to improve classification.

Eventually, polypeptides/patterns that give rise to the most important data points for prediction could be identified using a tandem mass spectrometry approach. Once a pattern is discovered, separations will be optimized to increase the amount of information about the polypeptides of interest, by slowing down separations during the elution of those polypeptides and speeding it up elsewhere. This would allow for the use of a separate, efficient assay for every diagnostic developed.

It is to be understood that the above embodiments are illustrative and not restrictive. The scope of the invention should be determined with respect to the scope of the appended claims, along with their full scope of equivalents.

## Claims

1. A method of performing analysis in a mass spectrometry system comprising:
(a) inputting a plurality of case and control samples;
(b) identifying any pattern or patterns of markers associated with said cases and any pattern or patterns of markers associated with said controls;
(c) among said markers identified in said cases and said controls, identifying at least 15 selected polypeptide markers that distinguish said cases from said controls in a mass spectrometry system; and
(d) performing an assay on a selected sample in a mass spectrometer by evaluating said at least 15 markers, and characterizing said selected sample based on said assay.

2. A method comprising:
a) collecting more than 10 case samples representing a clinical phenotypic state and more than 10 control samples representing patients without said clinical phenotypic state;
b) using a mass spectrometry platform system to obtain mass spectral data in said case samples and in said control samples without regard to a specific identity of at least some of said spectral components;
c) identifying representative patterns of selected markers that distinguish datasets between case samples and control samples; and
d) providing diagnostic products using said representative patterns

3. A method as claimed in claim 1 or claim 2 wherein the number of said selected markers is either more than 30, more than 50, more than 100, or more than 1000, optionally wherein the identity of at least some of said selected markers is not known.

4. A method as claimed in claim 2 wherein said product uses said representative patterns to identify said phenotypic state in additional samples with a disposable device.

5. A method as claimed in claim 2 or claim 4 wherein said diagnostic product comprises a mass spectrometry system to identify said representative states in patient samples.

6. A method as claimed in claim 2 or claim 4 wherein said diagnostic products use said mass spectrometry platform, e.g. a time of flight mass spectrometer, optionally wherein said diagnostic products are provided with a disposable microfluidics device, said disposable microfluidics device processing diagnostic samples for use in said mass spectrometry platform.

7. A method of performing an assay in a mass spectrometry system comprising:
(a) inputting a plurality of case samples and control samples;
(b) identifying any pattern or patterns of markers associated with said case samples and any pattern or patterns of markers associated with said control samples;
(c) among said markers identified in said case samples and said control samples, identifying patterns of at least selected markers that are present in both said cases and said controls;
(d) removing at least some of said selected markers from said case samples and said control samples;
(e) performing an assay on a selected sample by associating said selected sample as associated with said cases or said controls by reference to the remaining markers;
optionally wherein said assay on said selected sample further comprises separation of the remaining markers.

8. A method as claimed in claim 7 wherein said assay further comprises the step of performing a microfluidic separation on said selected sample.

9. A method as claimed in claim 7 or claim 8 wherein said removing step is performed in a microfluidics device, optionally a disposable microfluidics device.

10. A method as claimed in claim 7 wherein said removal step is performed in a solid phase extraction resin and/or a reversed phase chromatography resin.

11. A method as claimed in any preceding claim wherein the identity of at least some of said markers is not known.

12. A method as claimed in any preceding claim wherein said markers are polypeptides, e.g. proteins and/or wherein said samples contain complex mixtures of polypeptides.

13. A method of performing analysis in a mass spectrometry system comprising:
(a) performing sample preparation on a first sample in said mass spectrometry system;
(b) inputting said first sample to a mass spectrometer; and
(c) analyzing data from said mass spectrometry system in a data analysis system while a second sample is processed in said mass spectrometry system, wherein said mass spectrometry system is used to separate case samples from control samples in a diagnostic assay.

14. A method as claimed in any preceding claim wherein more than 50 preferably more than 100 of each of said case samples and control samples are used.

15. A method as recited in any preceding claim wherein said case samples represent a phenotypic state selected from a drug response or a drug resistance phenotype, a disease stage phenotype, a disease recurrence phenotype, a disease state phenotype, a treatment selection phenotype, a disease diagnostic phenotype, a drug toxicity phenotype, and an adverse drug response phenotype.

16. A method of analyzing biological samples comprising:
(a) inputting a sample or samples to a microfluidics electrophoretic and sample preparation device and applying pressure to said sample after at least partially preparing said sample in said sample preparation device;
(b) passing said sample to a mass spectrometer; and
(c) analyzing said sample,
optionally wherein said microfluidics device is a disposable device, further optionally wherein said samples comprise case samples and control samples and said method identifies more than 15 protein markers separating said case and control samples.

17. A method as claimed in any preceding claim wherein said sample or samples are detected using a time of flight mass spectrometer.

18. A method as claimed in any preceding claim wherein preparation of said sample or samples is performed in a microfluidics device, optionally a disposable microfluidics device.

19. A method as claimed in claim 18 wherein said microfluidics device comprises a separations device, optionally wherein said microfluidics device removes high abundance common proteins, further optionally wherein said microfluidics device comprises an electrospray source.

20. A method as claimed in any preceding claim wherein said method further comprises the step of performing analysis on additional selected samples.

21. A method as claimed in any preceding claim wherein data from one of said samples are being processed computationally while data from another sample is being obtained by mass spectrometry.

22. A method as claimed in any preceding claim wherein said method is performed to analyze at least 15 polypeptide markers.

23. A method as claimed in any preceding claim wherein data obtained from said method are used to separate and identify a disease state selected from the group consisting of a cancer disease state, a cardiovascular disease state, an infectious disease state, and a pregnancy-related disorder.

24. A system for analyzing biological samples comprising:
(a) a microfluidics device comprising a separation section and an electrospray section coupled to said separation section, said microfluidics device being a disposable device; and
(b) a mass spectrometer coupled to said microfluidics device,
optionally wherein said microfluidics device is a disposable device removably coupled to said mass spectrometer, further optionally wherein said microfluidics device comprises a capillary electrophoresis device.

25. A system as claimed in claim 24 wherein said microfluidics device comprises a solid phase extraction resin and/or a reverse phase chromatography device.

26. A system for analyzing biological samples comprising:
a) a sample preparation device comprising a separation section, a sample ionizer, and an ion excitation section;
b) a mass spectrometer coupled to said sample preparation device; and
c) a switch coupled to said ion excitation element,
optionally wherein said switch controllably excites samples to detect selected protein fragments.

27. A system for analyzing biological samples comprising:
a) a sample preparation device, said sample preparation device comprising a polypeptide denaturation system and a polypeptide removal system;
b) a sample analysis device comprising a mass spectrometer, said sample analysis device identifying at least some biological markers of interest in said biological samples that were bound to the polypeptides that were removed in said polypeptide removal system,
optionally wherein said polypeptide denaturation system is an acidification system.

28. A system as claimed in claim 27 wherein said denaturation system and said removal system are in a microfluidics device, optionally a disposable microfluidics device.

29. A system for analyzing biological samples comprising:
(a) a sample processing system comprising:
(i) a sample preparation device; and
(ii) a mass spectrometer coupled to said sample preparation device; and
(b) an analysis system coupled to said sample processing system, said analysis system detecting at least one common calibrant; comparing a time of detection to of said marker to a known time; and adjusting a speed of operation of said sample processing system in response to said comparing step,
optionally wherein said speed is adjusted to provide less precision in non-informative spectral regions and/or to provide greater precision in informative spectral regions.

30. A system for analyzing biological samples comprising:
(a) a sample processing system comprising:
(i) a sample preparation device; and
(ii) a mass spectrometer coupled to said sample preparation device; and
(b) an analysis system coupled to said sample processing system, said analysis system adjusting a speed of operation of said sample processing system at a time when a selected marker is expected to be detected,
optionally wherein said adjusting is a speeding adjustment for a component of less interest, or wherein said adjusting is a slowing adjustment for a marker of greater interest.

31. A system as claimed in claim 30 wherein said system is slowed at an expected time of detection of a marker of interest, and/or wherein said system compares a time of detection for a calibrant marker.

32. A method as claimed in claim 30 wherein said speed of operation is adjusted through a selected one of a temperature, a pressure, a voltage, or a current.

33. A system for analyzing biological samples comprising:
(a) an integrated sample preparation system and electrospray device; and
(b) a mass spectrometer adapted to receive samples from said electrospray device wherein said sample preparation and electrospray device is a disposable device to be coupled to said mass spectrometer,
optionally wherein said integrated sample preparation and electrospray device comprise a solid phase extraction resin, and/or a reversed phase chromatography device.

34. A system as claimed in any one of claims 24 to 33 wherein said mass spectrometer is a time of flight mass spectrometer.

35. A system as claimed in any one of claims 24 to 34 wherein said system analyzes at least 15 polypeptide markers and/or wherein said system is used to separate a disease state selected from the group consisting of a cancer disease state, a cardiovascular disease state, an infectious disease state, and pregnancy related disorders.

36. A system as claimed in any one of claims 26 to 35 wherein said sample preparation device is a disposable microfluidics device.

37. A system as claimed in any one of claims 24 to 36 wherein data from said mass spectrometer are used to separate a disease state selected from the group consisting of a cancer disease state, a cardiovascular disease state, an infectious disease state, and pregnancy related disorders.
